# EUROPEAN PATENT APPLICATION

(11) **EP 1 537 881 A1**
(43) Date of publication of application: **08.06.2005**
(21) Application number: 04257470.7
(22) Date of filing: 01.12.2004
(51) Int. Cl.: A61L 2/26, G06M 1/00

(54) **Use indicator system for medical device**

(30) Priority: 06.12.2003 US 527629 P; 16.11.2004 US 989580
(71) Applicant: LINVATEC CORPORATION, Largo, Florida 33773 (US)
(72) Inventor: Bogren, Robert G, Tampa, Florida 33626 (US); Fagan, Marianne, Largo, Florida (US); Aday, Jorge L, Florida 33772 (US)
(74) Representative: Gambell, Derek

(57) **Abstract**

A cycle counter for sterilization cycles for a disposable instrument features a plurality of tabs. With each sterilization after the initial use, a tab is removed by medical personnel. The instrument is disposed of after the final use with no tabs left.

## Description

### BACKGROUND OF THE INVENTION

### FIELD OF THE INVENTON

The field of the invention is sterilization cycle counting for a disposable medical instrument.

This system enables a user to track the number of uses of a reusable medical device. This system incorporates sterilization indicator tabs that are added to a portion of the plastic hub of reusable arthroscopic shavers and burrs. The invention was created to develop a method that could be used by the operating room staff which would indicate how many times a reusable shaver blade has been cycled. Without such a system, the operating room personnel have no way of knowing how many times the device has been cycled; i.e. used and sterilized.

A typical cycle of use for a reusable blade or burr would be as follows:
1) Product is delivered, sealed and sterilized, to the hospital.
2) Product is used for surgery.
3) The shaver is cleaned at the hospital site using common cleaning techniques.
4) The device is then autoclaved (or otherwise sterilized) along with other reusable devices.
5) The device is delivered acseptically back to the surgical suite.

With conventional arthroscopic/endoscopic shavers there is no visual indicator of how many times the device has been through this cycle. It has been reported that some devices have been used well beyond the recommended useful life. One such device is described by its manufacturer to be qualified for a given number of uses, for example, seven. The effectiveness of the device beyond this point is not recommended and the manufacturer advises that the device be disposed of after the recommended number of cycles.

### SUMMARY OF THE INVENTION

A cycle counter for sterilization cycles for a disposable instrument features a plurality of tabs. With each sterilization, after the initial use, a tab is removed by medical personnel. The instrument is disposed of after the final use with no tabs left.

### BRIEF DESCRIPTION OF THE DRAWINGS

Figure 1 is an exploded side elevational view of a prior art shaver blade.

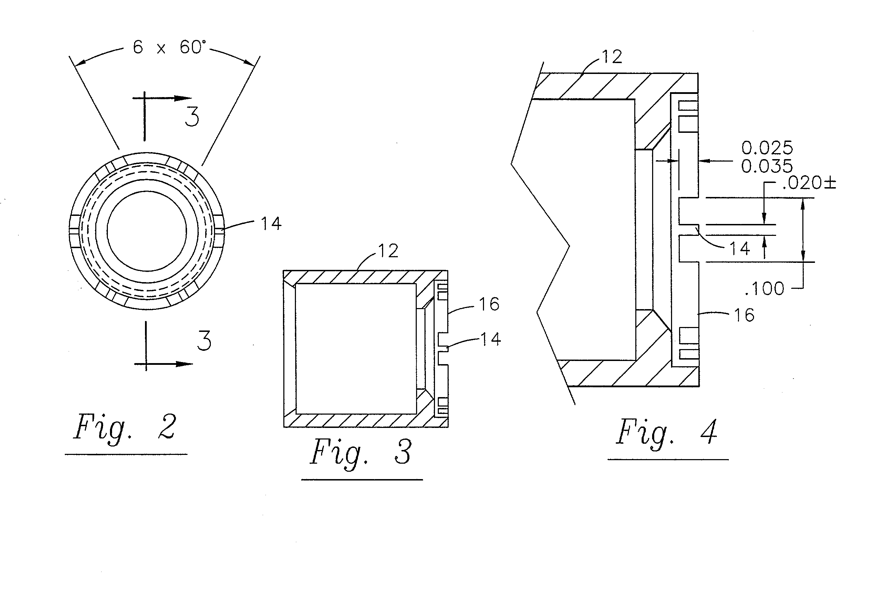

Figure 2 is an end view of a component of the shaver blade of Figure 1, modified in accordance with the principles of this invention.
Figure 3 shows a cross-section of Figure 2 taken along the line A-A.
Figure 4 is an enlarged view of a portion of Figure 3.

### DESCRIPTION OF THE PREFERRED EMBODIMENT

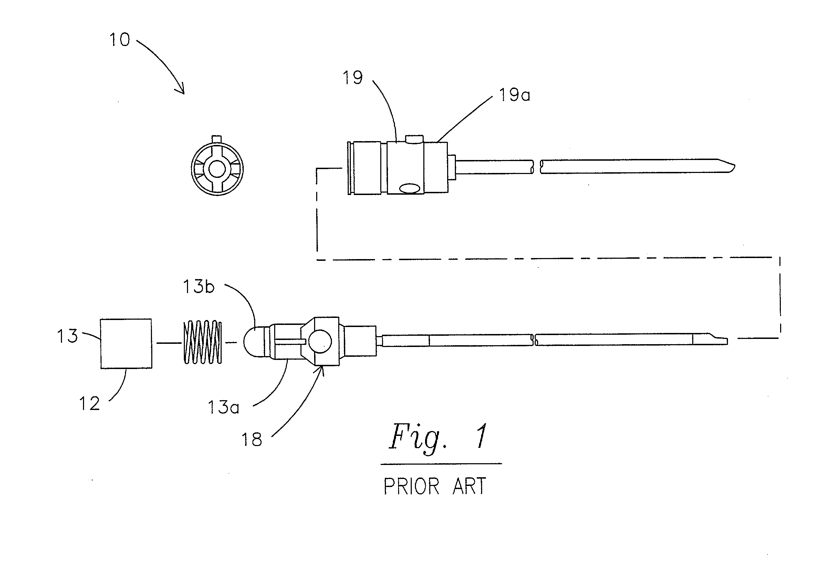

As shown in Figure 1, a prior art shaver blade 10 comprises an inner member 18 rotatably situated within an outer member 19. The outer member has a molded hub 19a at its proximal end to facilitate its attachment to a handpiece (not shown). Inner member 18 has a conventional spring retainer 12 which is press fit adjacent a ring tang 13b to facilitate the retention of the shaver blade within the handpiece in a manner enabling rotation of the inner member. Retainer 13 is situated adjacent a hub 13a of the inner member. In the prior art device the proximal rim 13 of retainer 12 is smooth and circular. In the preferred embodiment of the invention, the hubs 13a and 19a as well as components 12 and 13b are molded from suitable polymeric material and rim 13 is modified to form new rim 16 having tabs 14. The sterilization indicator tabs 14 provide a simple, visual, inexpensive method of indicating how many times the shaver blade has been cycled. If the number of recommended uses is seven, six small, removable sterilization indicator tabs 14 are molded into the distal rim 16 of plastic spring retainer component 12 formed. These tabs are easily removable and, if they are removed by the user one at a time after each sterilization, they become visual indicators for the operating room personnel of how many times the device has been cycled. These tabs are an integral feature of the device and their removal does not adversely affect the performance of the device in any way.

It will be understood that the tabs may be attached to or integrally formed with other portions of the hubs of the inner or outer members. For example, the tabs could be formed as extensions from the proximal or distal ends of hubs 13a or 19a.

The new processing cycle would be as follows:
1) Product is delivered, sealed and sterilized, to the hospital, with 6 sterilization indicator tabs.
2) Product is used for surgery.
3) The shaver is cleaned at the hospital site using common cleaning techniques.
4) One of the sterilization indicator tabs is removed from the device using a common surgical tool such as a forceps.
5) The device is then autoclaved along with other reusable devices.
6) The shaver is delivered acseptically back to the surgical suite for reuse.
7) The cycle is repeated until all tabs have been removed after which the device is disposed of.

It will be understood that any number of tabs may be molded into rim 16, depending upon how many uses one desires to track.

It will be understood by those skilled in the art that numerous improvements and modifications may be made to the preferred embodiment of the invention disclosed herein without departing from the spirit and scope thereof.

## Claims

1. An apparatus for tracking the number of sterilization cycles for a medical instrument, comprising:
a sterilization cycle counter that gives a visual indication of the number of remaining uses without moving parts or reliance on chemical reaction.

2. The apparatus of claim 1, wherein:
said counter is not sensitive to heat or pressure cycles from autoclaving.

3. The apparatus of claim 2, wherein:
said counter comprises a plurality to tabs.

4. The apparatus of claim 3, wherein:
said tabs are selectively removable.

5. The apparatus of claim 4, wherein:
one tab is removed by medical personnel for each sterilization.

6. The apparatus of claim 5, wherein:
the removal of the last tab indicates the final sterilization.

7. The apparatus of claim 6, wherein:
the tabs are removable, one at a time, with a surgical tool.

8. The apparatus of claim 7, wherein:
forceps are used to remove each tab.

9. The apparatus of claim 8, wherein:
for n cycles of the instrument including the initial use, n-1 tabs are provided.

10. The apparatus of claim 1 wherein the apparatus is a shaver blade and the sterilization cycle counter comprises a plurality of removable tabs secured to a component of the shaver blade.

11. The apparatus of claim 10 wherein said component is the hub of the shaver blade.

12. The apparatus of claim 10 wherein said component is a spring retainer.

13. A method of keeping track of sterilization cycles for a medical instrument, comprising:
providing a tab for each remaining sterilization cycle;
removing said tab in close proximity in time to said sterilization;
disposing of the instrument after use, when no tab remains.

14. The method of claim 13, comprising:
providing n-1 tabs for an instrument that can be used n times, including the initial use.

15. The method of claim 14, comprising:
removing a tab with forceps.
